# EUROPEAN PATENT SPECIFICATION

(11) **EP 2 443 101 B1**
(45) Date of publication and mention of the grant of the patent: **27.03.2019**
(21) Application number: 10790324.7
(22) Date of filing: 21.06.2010
(51) Int. Cl.: C07D 265/34

(54) **BAIT CHEMISTRIES IN HYDROGEL PARTICLES FOR SERUM BIOMARKER ANALYSIS**
KÖDERCHEMIKALIEN IN HYDROGELPARTIKELN FÜR DIE SERUM-BIOMARKER-ANALYSE
COMPOSITIONS CHIMIQUES D'APPÂTS DANS DES PARTICULES D'HYDROGEL POUR UNE ANALYSE DE BIOMARQUEURS SÉRIQUES

(30) Priority: 19.06.2009 US 218670 P
(43) Date of publication of application: 25.04.2012
(73) Proprietor: Liotta, Lance, Bethesda, MD 20817 (US); Espina, Virginia, Rockville, MD 20853 (US); Luchini, Alessandra, Burke, VA 22015 (US); Tamburro, Davide, Bristow, VA 20190 (US)
(72) Inventor: Liotta, Lance, Bethesda, MD 20817 (US); Espina, Virginia, Rockville, MD 20853 (US); Luchini, Alessandra, Burke, VA 22015 (US); Tamburro, Davide, Bristow, VA 20190 (US)
(74) Representative: HGF Limited
(86) International application number: PCT/US2010/039357
(87) International publication number: WO 2010/148397

(56) References cited:
- US-A1- 2003 185 793
- US-A1- 2006 088 476
- US-A1- 2007 092 973
- US-A1- 2007 142 560
- US-A1- 2008 090 737
- US-A1- 2009 044 603
- US-A1- 2009 148 961

## Description

### FIELD OF THE INVENTION

The invention pertains to the incorporation of dye affinity baits into hydrogel particles used to concentrate, purify and protect small and labile analytes from degradation in blood and other body fluids. This invention describes the identification of novel non-triazine organic dye chemistries that can be used as affinity baits to capture proteins and other bio molecules useful in the fields of medical diagnostics, environmental science, toxicology, and infectious disease. Incorporation of unique affinity dye compounds within hydrogel capture particles improves analyte yield and preanalytical precision, and stabilizes the analyte against degradation, while increasing measurement sensitivity. The particles in this invention can be used for routine clinical testing as well as for discovery of low abundance disease biomarkers. Example hydrogel particles containing new high affinity bait chemistries were used to identify a new set of human serum biomarkers.

### BACKGROUND OF THE INVENTION

There has recently been a surge of interest in the value and clinical potential of proteomic biomarkers. A general belief in the medical community is that the earlier a disease is treated, the more successful the therapeutic outcome. Consequently, the routine clinical availability of biomarker tests specific for early-stage diseases has tremendous potential to dramatically improve public health, even using currently utilized therapeutic modalities. For example, clinical oncologists expect that biomarker detection of pre-metastatic solid tumors of the breast, lung, ovary, and colon could lead to a significant improvement in survival. Unfortunately, despite the urgent clinical need, in the past ten years, for all disease categories combined, only a handful of novel biomarkers have graduated to routine clinical use. The slow biomarker pipeline persists despite considerable efforts within diagnostics research. The reasons for this failure stem from fundamental technical and biologic roadblocks spanning the biomarker development pipeline from biomarker identification and measurement to initial clinical validation. Two of these major roadblocks are:
*Low Abundance:* Disease-relevant biomarkers may exist in exceedingly low concentrations within a complex mixture of body fluid proteins containing high abundance proteins such as albumin.
*Instability:* Immediately after the blood or other body fluid is collected (e.g. by venipuncture), degradation of proteins can occur, which is mediated by endogenous or exogenous proteinases.

US 2008/0090737 A1 Boschetti discloses a method of decreasing the concentration of more abundant analyte species in a complex sample relative to the less abundant analyte species, but at the same time not reducing the diversity of the analytes in the resultant sample following treatment. The method employs particles which carry binding moieties which are selected from peptides, oligonucleotides, oligosaccharides, antibodies or aptamers.

### SUMMARY OF THE INVENTION

Accordingly, the present invention provides a chemical affinity bait as set forth in any of claims 1 to 13.

As described herein hydrogel particles are used to concentrate, partially purify and protect small and labile analytes from degradation in blood and other body fluids. Protein analytes extracted from the particles are analyzed by ELISA, western blotting, reverse phase protein arrays, and mass spectrometry. Also described are bait strategies that can extend the classes of analytes captured by the particles and a workflow involving N-isopropylacrylamide particles with a set of affinity bait examples, gel electrophoresis results for common protein biomarkers as well as mass spectrometry for the screening of sera from patient.

### BRIEF DESCRIPTION OF THE DRAWING

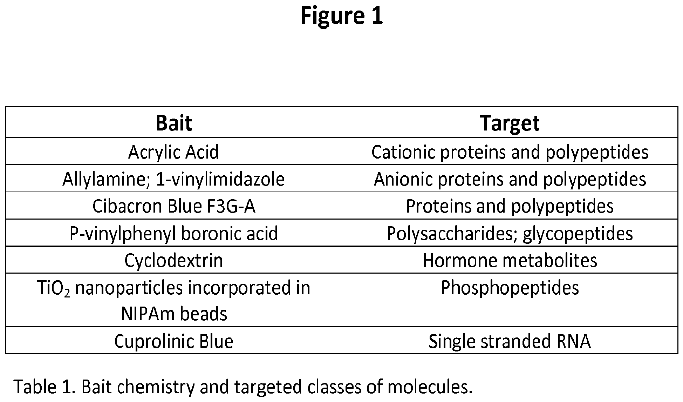

Figure 1. A general description of bait chemistries and corresponding target classes of molecules.

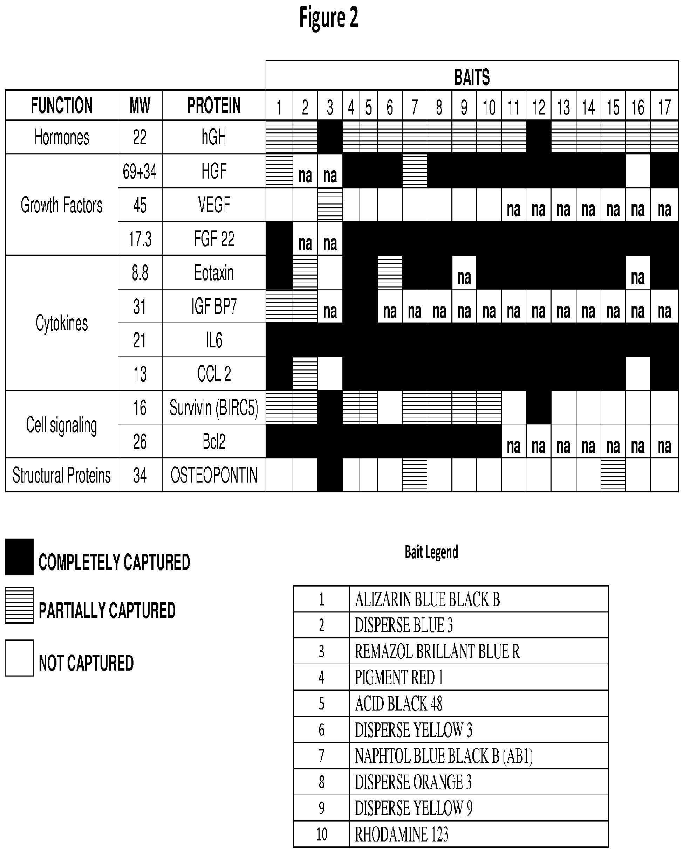

Figure 2a -2b. Low abundance clinical protein biomarkers captured and enriched by particles. The table was constructed by incubating clinically relevant low abundance labile proteins with hydrogel particles containing 17 different examples of unique affinity baits. SDS-PAGE and silver-staining experiments were used to determine the ability of each dye to bind these proteins. The included table indicates the affinity of each bait for specific proteins.

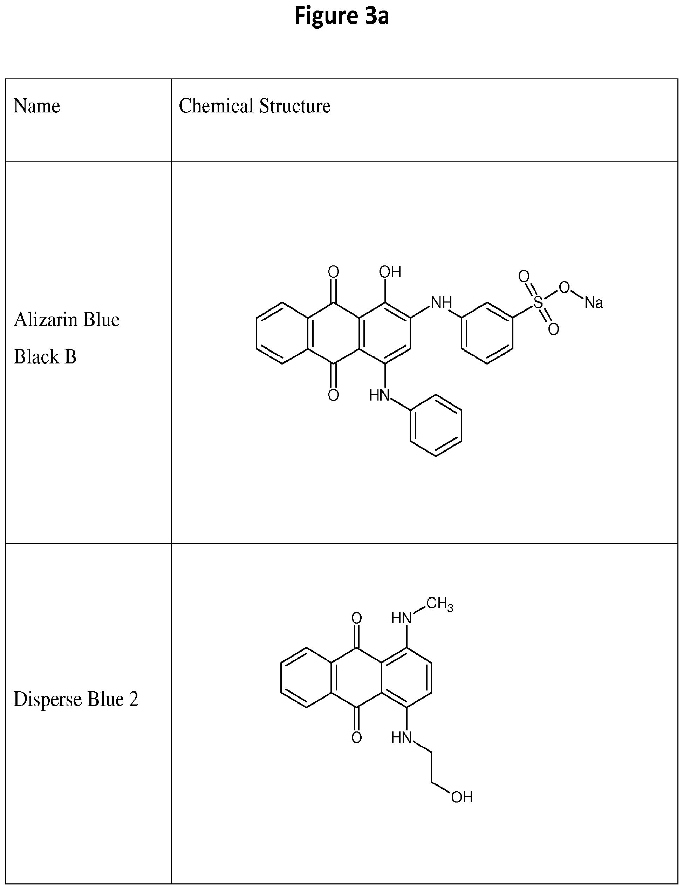

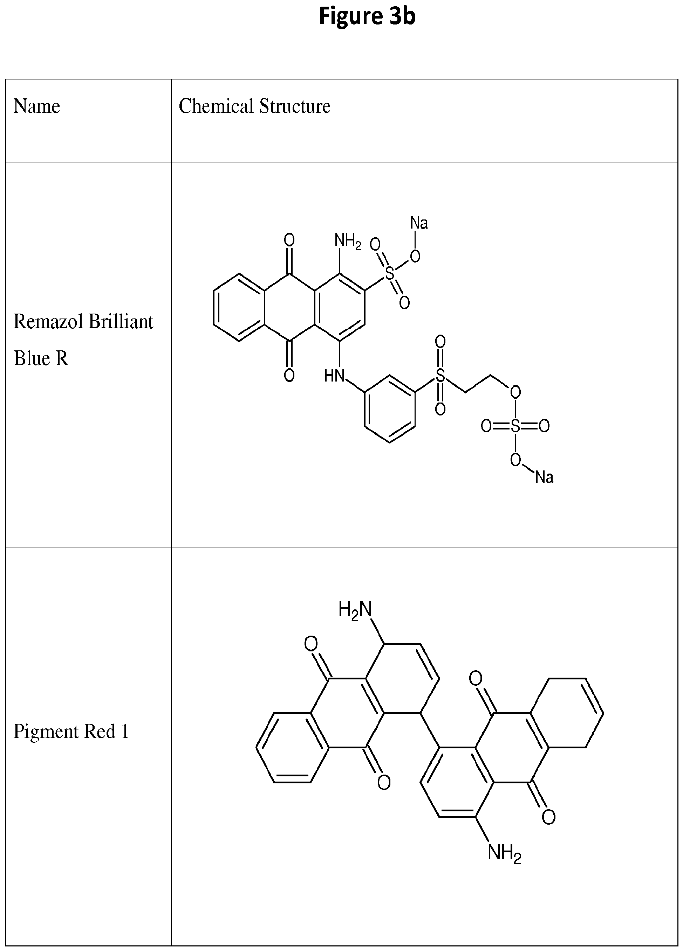

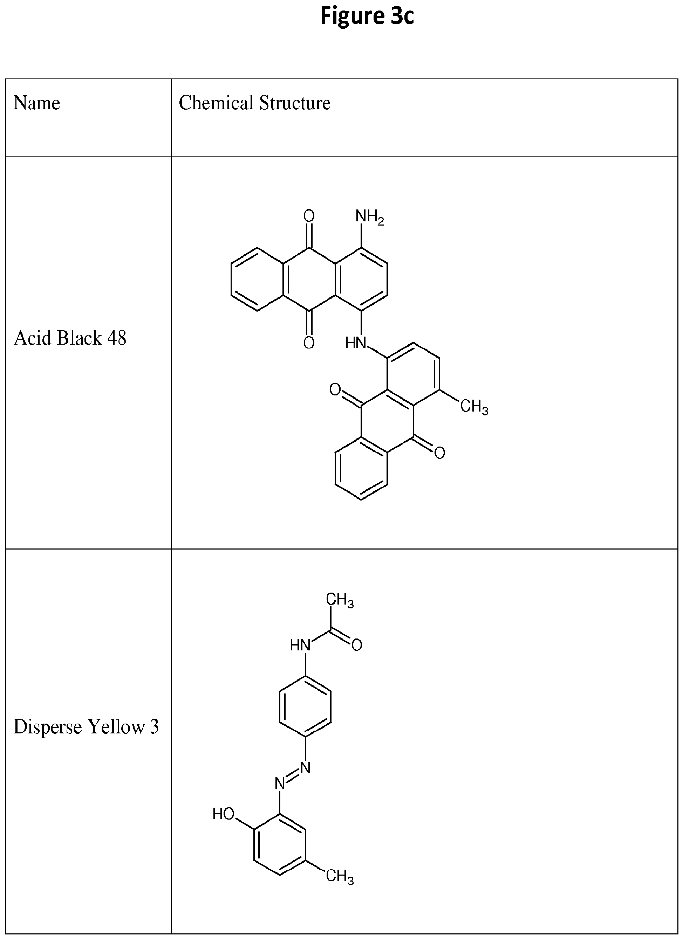

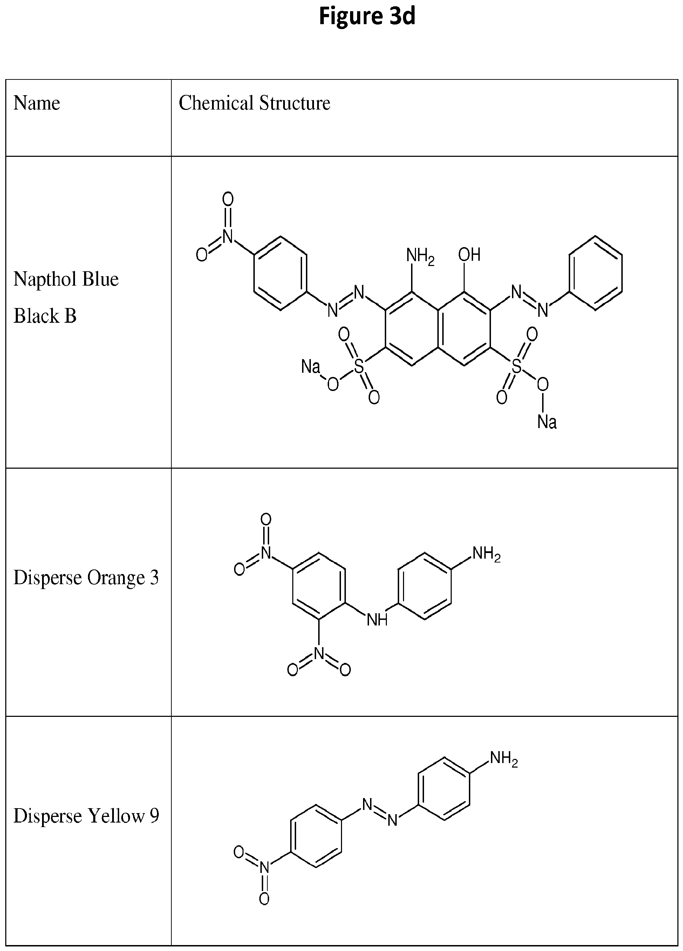

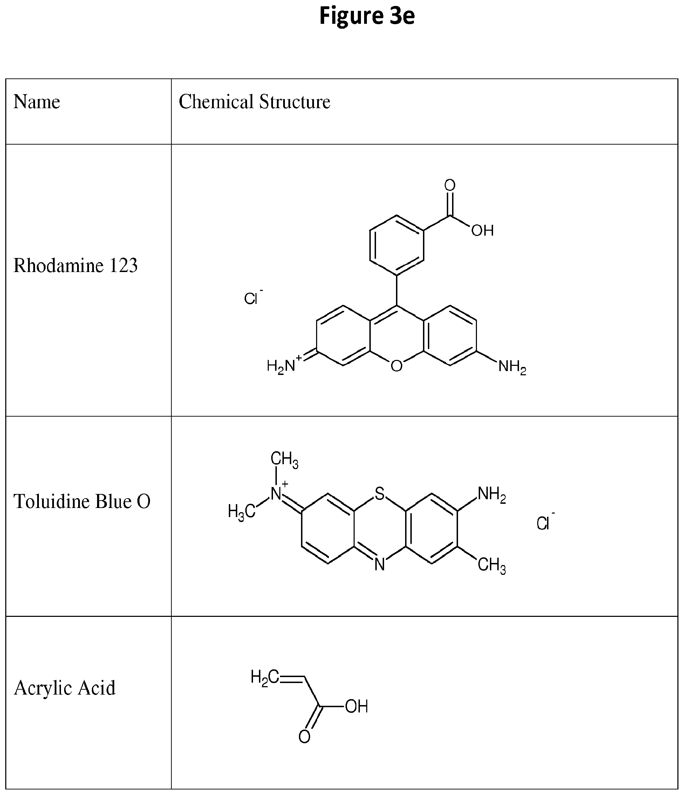

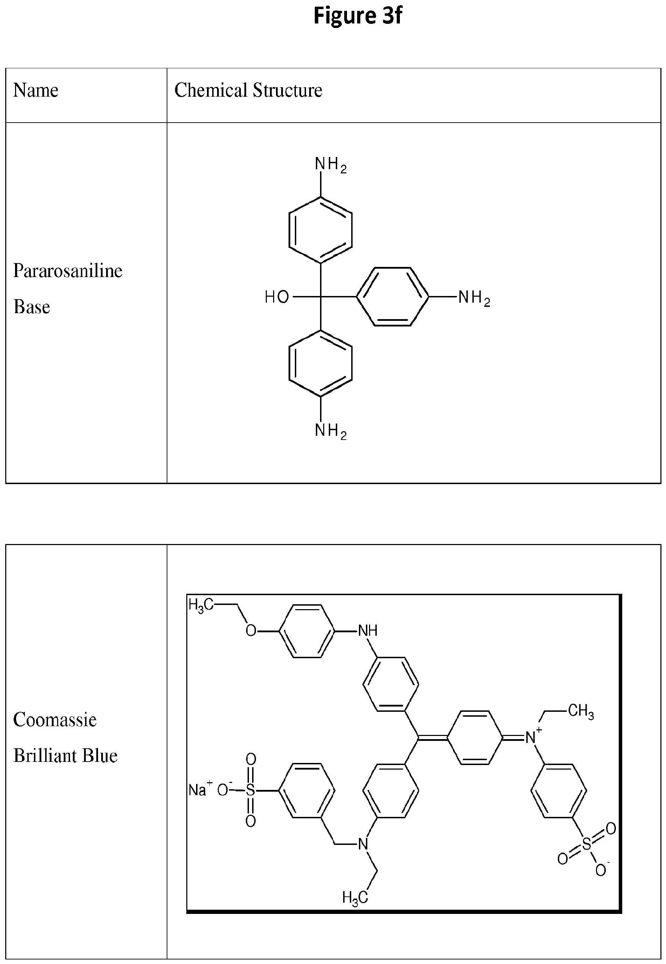

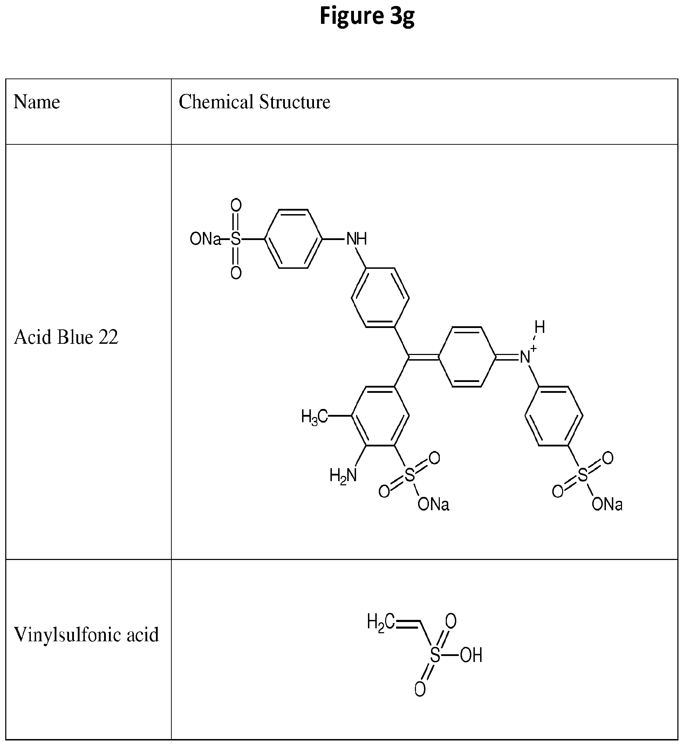

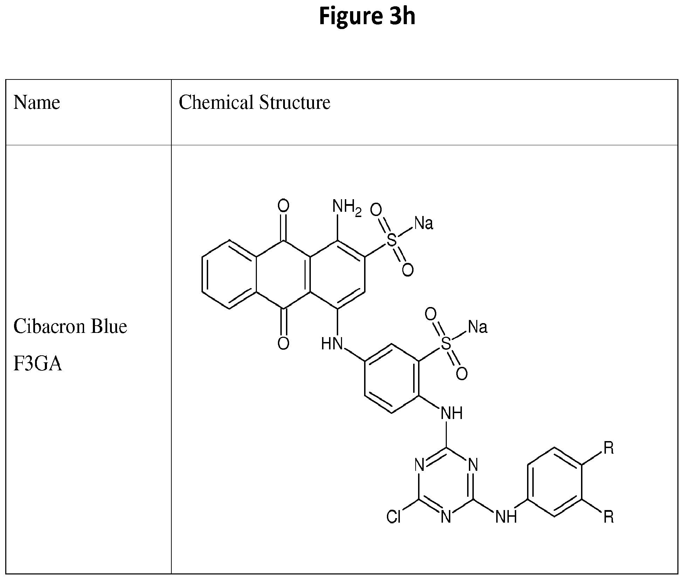

Figure 3a-3c.Unique set of chemical affinity baits incorporated in the particle matrices demonstrate affinity capture of a subset of analytes. Particles contain the following chemical affinity baits 1 Alizarin Blue Black B; 2 Disperse Blue 3; 3 Remazol Brillan Blue R; 4 Pigment Red 177; 5 Acid Black 48; 6 Disperse Yellow 3; 7 Naphtol Blue Black B (Ab1); 8 Disperse Orange 3; 9 Disperse Yellow 9; 10 Rhodamine 123; 11 Toluidine Blue O; 12 Acryic Acid, 13 Pararosaniline Base, 14 Brillant Blue R; 15 Acid Blue 22, 16 Vinyl Sulfonic Acid; 17 Cibacron Blue F3GA

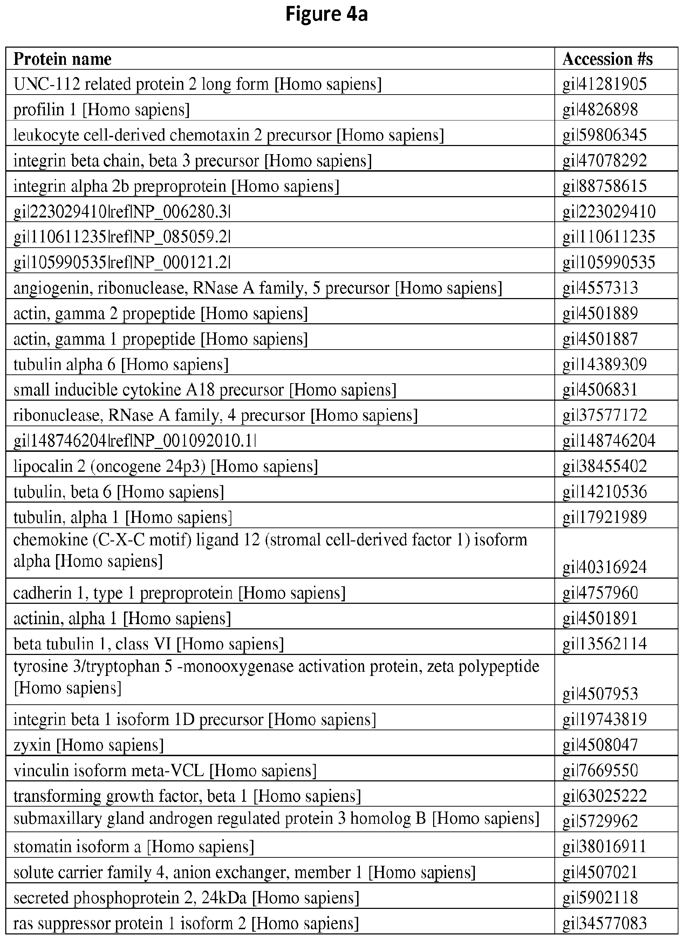

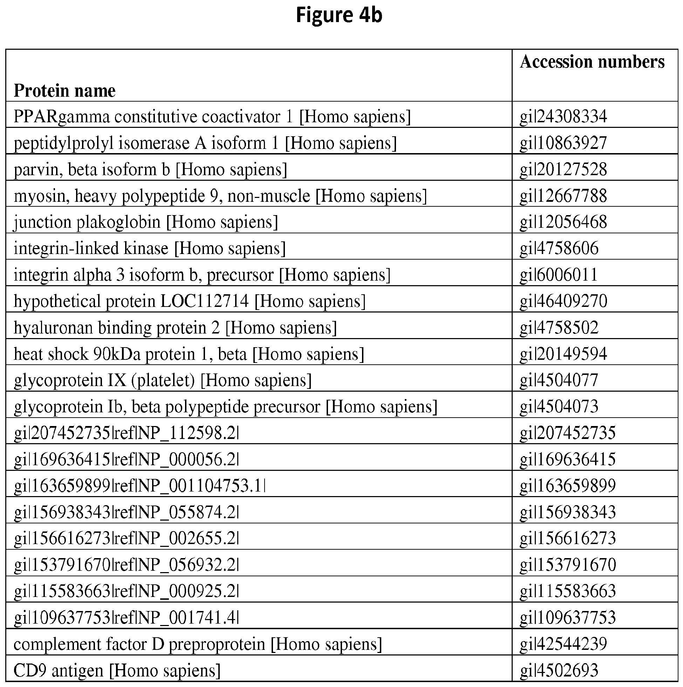

Figure 4a-4b: New proteins never sequenced in serum before (not present in the HUPO Plasma Proteome Project comprehensive list, ttp://www.hupo.org/research/hppp/) were sequenced by nanospray tandem mass spectrometry after serum processing with particles. particles carrying the following baits 1 Alizarin Blue Black B; 2 Disperse Blue 3; 3 Remazol Brillan Blue R; 4 Pigment Red 177; 5 Acid Black 48; 6 Disperse Yellow 3; 7 Naphtol Blue Black B (Ab1); 8 Disperse Orange 3; 9 Disperse Yellow 9; 10 Rhodamine 123; 11 Toluidine Blue O; 12 Acryic Acid, 13 Pararosaniline Base, 14 Brillant Blue R; 15 Acid Blue 22, 16 Vinyl Sulfonic Acid; 17 Cibacron Blue F3GA were used. Aliquots of 100 µL of nanoparticles (1 mg/mL) were incubated with 100 µL of serum for 30 minutes, separated by centrifugation and chemically eluted. Proteins listed in this table were found in at least one of the affinity bait loaded particles. All proteins shown were selected to have a p value (probability of randomized identification) <= 0.05.

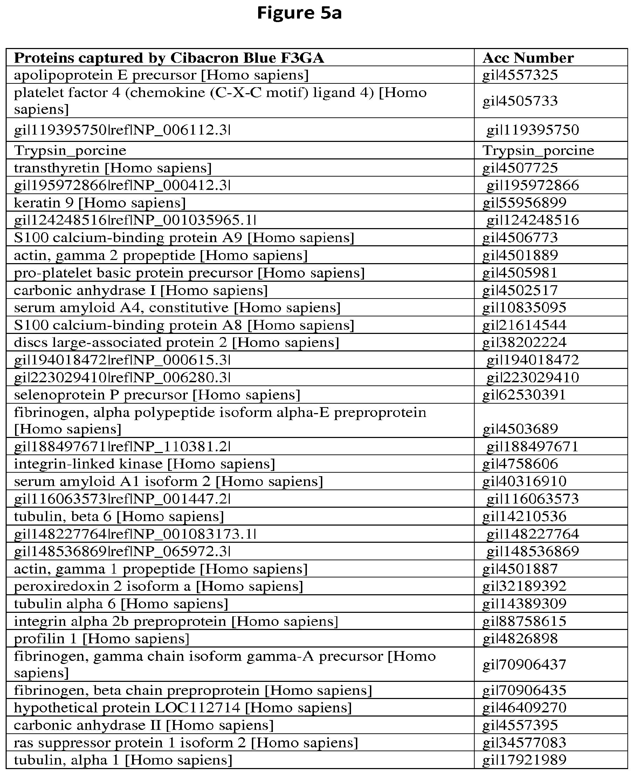

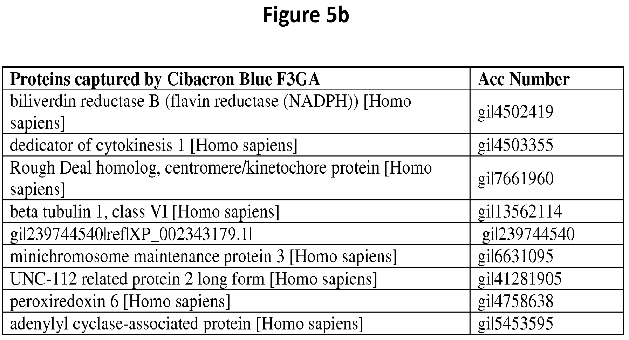

Figure 5: Protein sequenced by nanospray tandem mass spectrometry after serum processing with particles. Aliquots of 500 µL of particles (1 mg/mL) containing Cibacron Blue F3GA affinity bait were incubated with 500 µL of serum for 30 minutes, separated by centrifugation and eluted with 70% ACN - 10% ammonium hydroxide. Proteins listed in column 1 were found in the sample processed with Cibacron Blue F3GA functionalized particles and not in raw serum. All proteins shown are selected to have a p value (probability of randomized identification) <= 0.01.

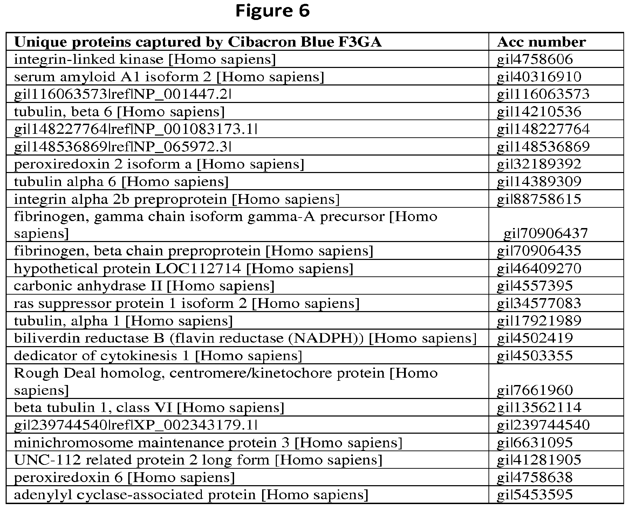

Figure 6: Protein sequenced by nanospray tandem mass spectrometry after serum processing with particles. Aliquots of 500 µL of particles (1 mg/mL) containing Cibacron Blue F3GA affinity bait were incubated with 500 µL of serum for 30 minutes, separated by centrifugation and eluted with 70% ACN - 10% ammonium hydroxide. Proteins listed in column 1 were found only in the Cibacron Blue F3GA loaded particles and not in the particles containing alternative chemical affinity dyes. All proteins shown are selected to have a p value (probability of randomized identification) <= 0.01.

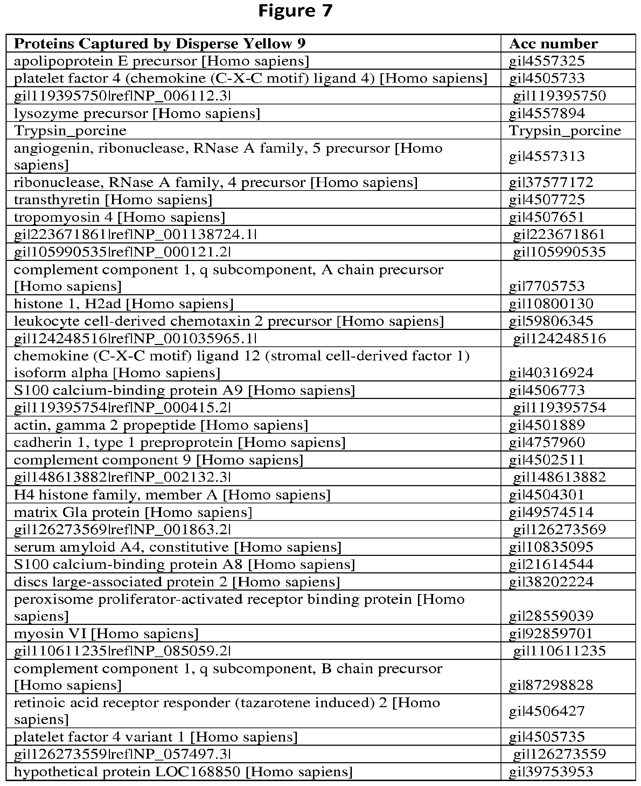

Figure 7: Protein sequenced by nanospray tandem mass spectrometry after serum processing with particles. Aliquots of 500 µL of particles (1 mg/mL) containing Disperse Yellow 9 affinity bait were incubated with 500 µL of serum for 30 minutes, separated by centrifugation and eluted with 70% ACN - 10% ammonium hydroxide. Proteins listed in column 1 were found in the sample processed with Disperse Yellow 9functionalized particles and not in raw serum. All proteins shown are selected to have a p value (probability of randomized identification) <= 0.01.

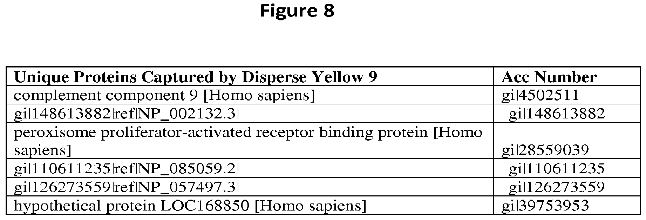

Figure 8: Protein sequenced by nanospray tandem mass spectrometry after serum processing with particles. Aliquots of 500 µL of particles (1 mg/mL) containing Disperse Yellow 9 affinity bait were incubated with 500 µL of serum for 30 minutes, separated by centrifugation and eluted with 70% ACN - 10% ammonium hydroxide. Proteins listed in column 1 were found only in the Disperse Yellow 9loaded particles and not in the particles containing alternative chemical affinity dyes. All proteins shown are selected to have a p value (probability of randomized identification) <= 0.01.

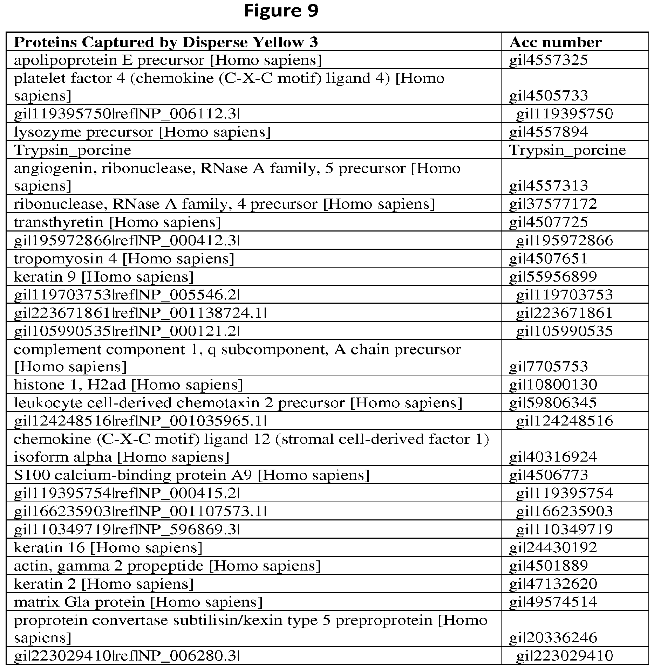

Figure 9: Protein sequenced by nanospray tandem mass spectrometry after serum processing with particles. Aliquots of 500 µL of particles (1 mg/mL) containing Disperse Yellow 3 affinity bait were incubated with 500 µL of serum for 30 minutes, separated by centrifugation and eluted with 70% ACN - 10% ammonium hydroxide. Proteins listed in column 1 were found in the sample processed with Disperse Yellow 3functionalized particles and not in raw serum. All proteins shown are selected to have a p value (probability of randomized identification) <= 0.01.

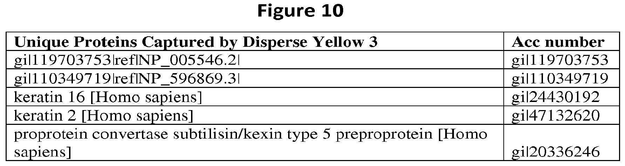

Figure 10: Protein sequenced by nanospray tandem mass spectrometry after serum processing with particles. Aliquots of 500 µL of particles (1 mg/mL) containing Disperse Yellow 3 affinity bait were incubated with 500 µL of serum for 30 minutes, separated by centrifugation and eluted with 70% ACN - 10% ammonium hydroxide. Proteins listed in column 1 were found only in the Disperse Yellow 3loaded particles and not in the particles containing alternative chemical affinity dyes. All proteins shown are selected to have a p value (probability of randomized identification) <= 0.01.

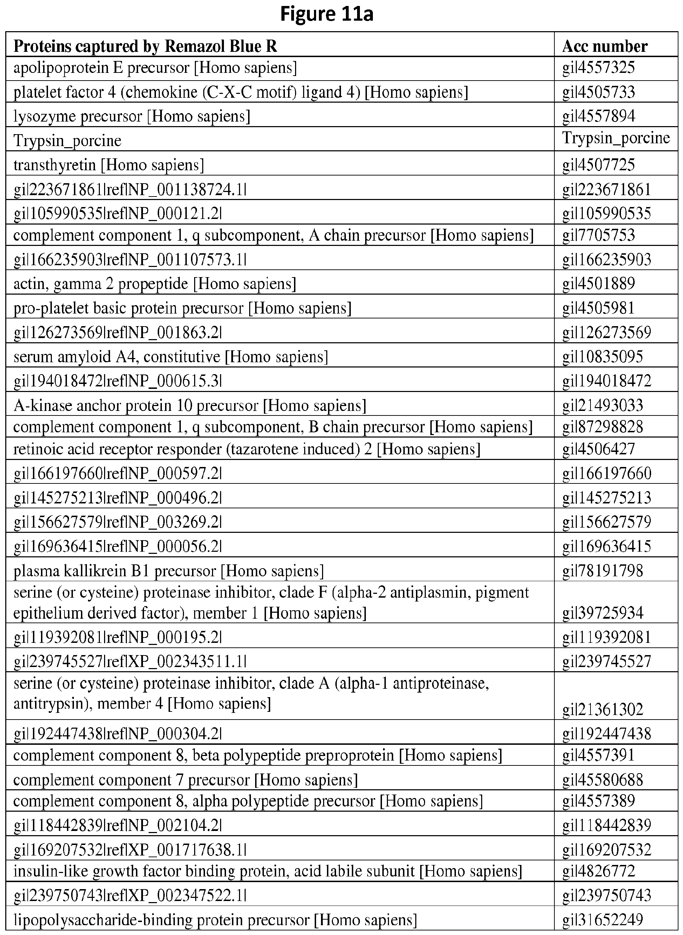

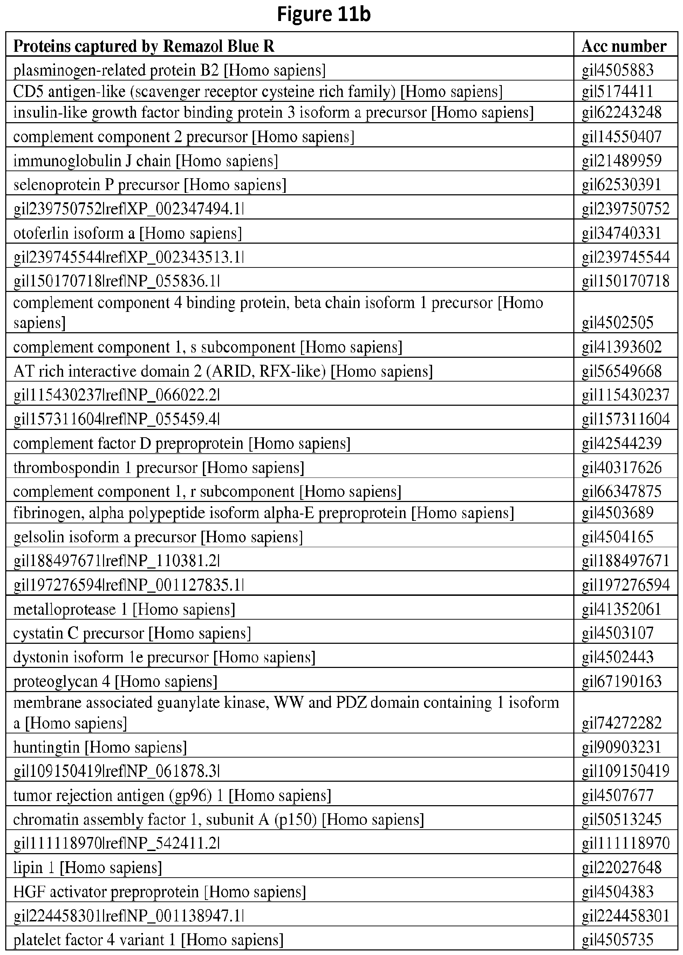

Figure 11a-11b: Protein sequenced by nanospray tandem mass spectrometry after serum processing with particles. Aliquots of 500 µL of particles (1 mg/mL) containing remazol brilliant blue R affinity bait were incubated with 500 µL of serum for 30 minutes, separated by centrifugation and eluted with 70% ACN - 10% ammonium hydroxide. Proteins listed in column 1 were found in the sample processed with remazol brilliant blue R functionalized particles and not in raw serum. All proteins shown are selected to have a p value (probability of randomized identification) <= 0.01.

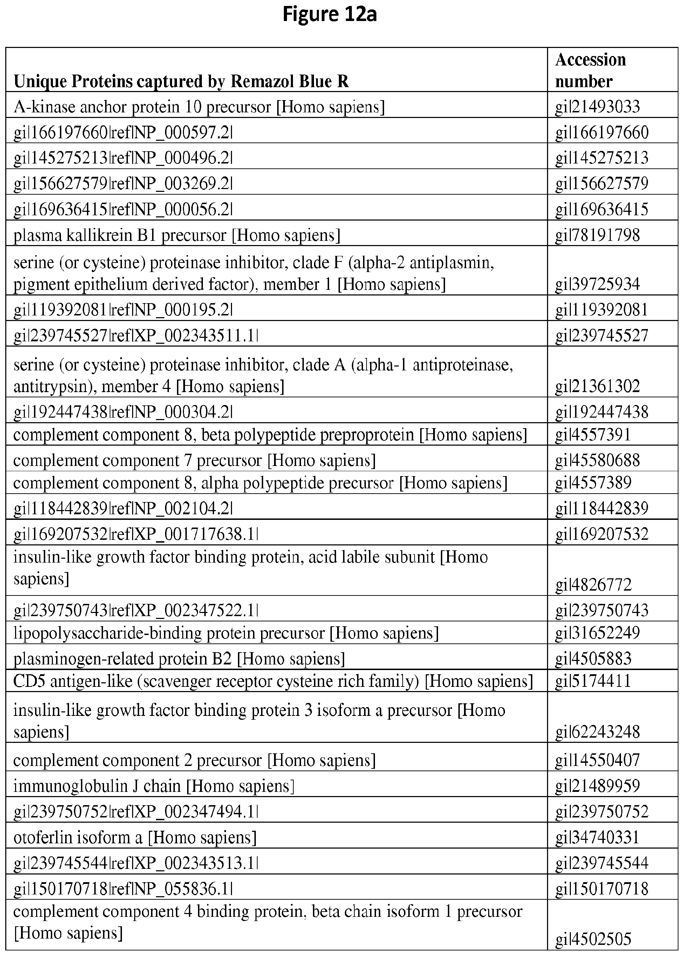

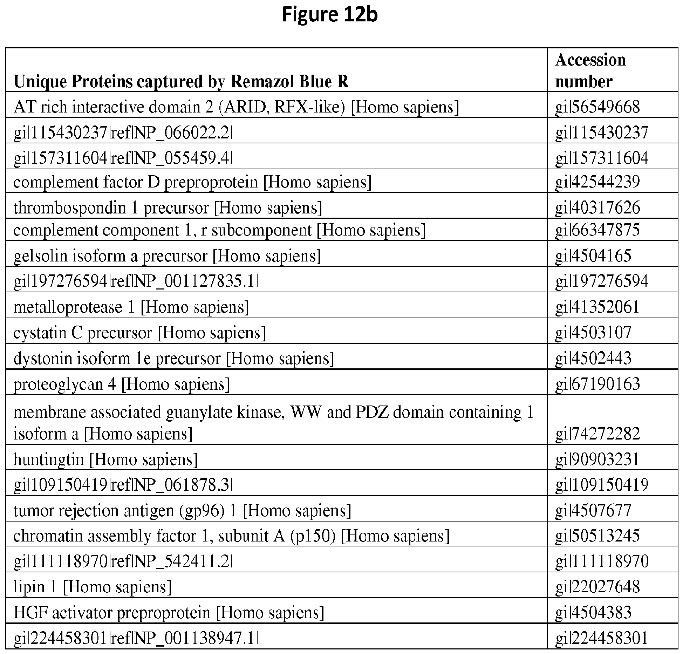

Figure 12a-12b: Protein sequenced by nanospray tandem mass spectrometry after serum processing with particles. Aliquots of 500 µL of particles (1 mg/mL) containing remazol brilliant blue R affinity bait were incubated with 500 µL of serum for 30 minutes, separated by centrifugation and eluted with 70% ACN - 10% ammonium hydroxide. Proteins listed in column 1 were found only in the remazol brilliant blue R loaded particles and not in the particles containing alternative chemical affinity dyes. All proteins shown are selected to have a p value (probability of randomized identification) <= 0.01.

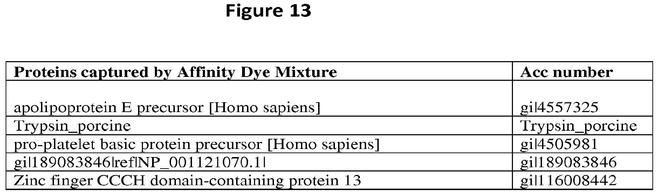

Figure 13: Protein sequenced by nanospray tandem mass spectrometry after serum processing with particles containing a mixture of remazol brilliant blue R, disperse yellow 3, and disperse yellow. Aliquots of 500 µL of particles (1 mg/mL) containing Cibacron Blue F3GA affinity bait were incubated with 500 µL of serum for 30 minutes, separated by centrifugation and eluted with 70% ACN - 10% ammonium hydroxide. Proteins listed in column 1 were found in the sample processed with the mixture of functionalized particles and not in raw serum. All proteins shown are selected to have a p value (probability of randomized identification) <= 0.01.

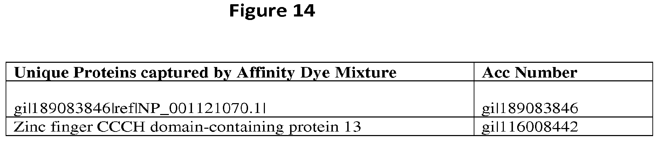

Figure 14: Protein sequenced by nanospray tandem mass spectrometry after serum processing with particles containing a mixture of remazol brilliant blue R, disperse yellow 3, and disperse yellow. Aliquots of 500 µL of particles (1 mg/mL) containing the mixture affinity bait were incubated with 500 µL of serum for 30 minutes, separated by centrifugation and eluted with 70% ACN - 10% ammonium hydroxide. Proteins listed in column 1 were found only in the mixture loaded particles and not in the particles containing alternative chemical affinity dyes. All proteins shown are selected to have a p value (probability of randomized identification) <= 0.01.

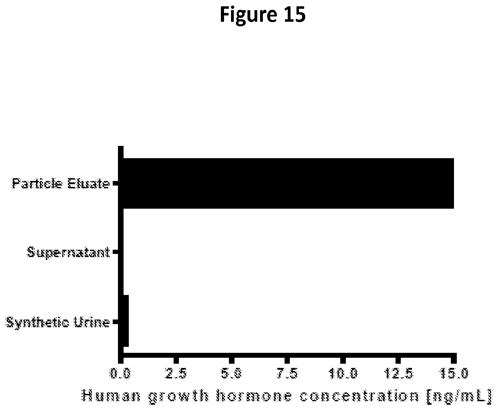

Figure 15: NIPAm/Remazol Brilliant Blue R particles, incubated with 30 mL of solution of human growth hormone (hGH) (0.242 ng/mL) in synthetic urine, sequestered all hGH present in solution and increased the concentration of hGH more than 60 times (14.9 ng/mL).

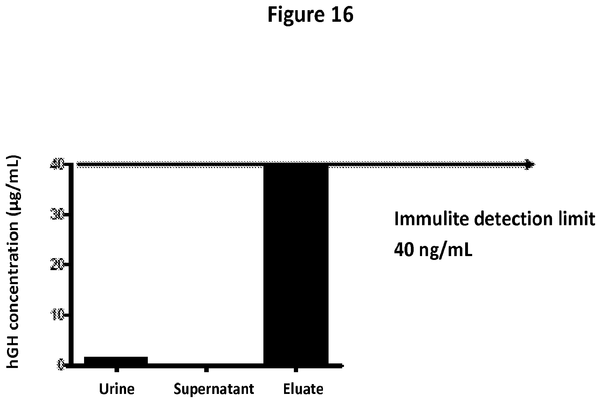

Figure 16. NIPAm/Acid Black 48 particles completely deplete the supernatant and amplify > 30 fold the concentration of hGH spiked in synthetic urine at a concentration of 1 ng/mL. Particles were incubated with the hGH solution, separated by centrifugation, and supernatant and particle content was measured by Immulite 1000.

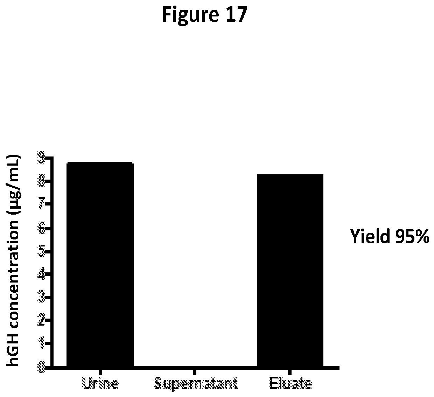

Figure 17. NIPAm/Acid Black 48 particles have a yield of 95% of recovering hGH in synthetic urine. Particles were incubated with 1 mL of synthetic urine containing 8.74 ng/mL hGH, separated by centrifugation, the particle content was eluted, reconstituted in the same starting volume. Initial solution and particle eluate were measured by Immulite 1000.

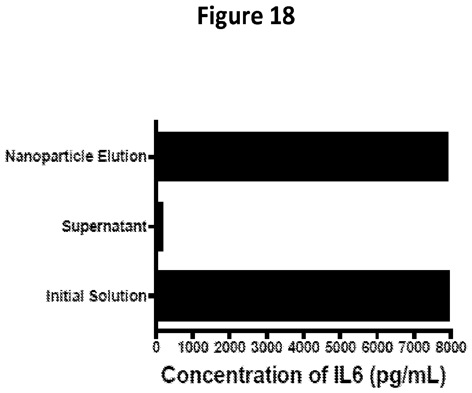

Figure 18: NIPAm/Disperse Yellow 9 particles have a yield of 95% of recovering interleukin 6 (IL 6) in synthetic perspiration. Particles were incubated with 50 µL of synthetic perspiration containing 7912.5 pg/mL IL6 and separated by centrifugation; the particle content was eluted and reconstituted in the same starting volume. IL6 concentration in the eluate was 7875 pg/mL. Initial solution and particle eluate were measured by Immulite 1000.

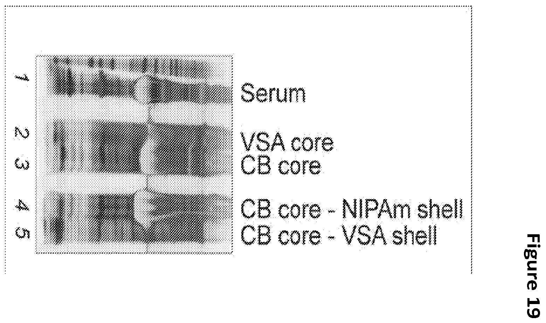

Figure 19: SDS PAGE analysis showed that particles functionalized with two affinity baits in the core and in the shell acquire a behaviour that is the sum of the two baits, and the external, shell bait does not interfere with the binding of proteins to the inner, core bait. Lane 1, serum; 2 vinyl sulfonic acid particles; 3 cibacron blue F3GA particles; 4 N-isopropylacrylamide shell-cibacron blue F3GA core particles; 5 cibacron blue F3GA core-vinylsulfonic acid shell particles.

### DETAILED DESCRIPTION OF THE PREFERRED EMBODIMENT(S)

The following examples are put forth so as to provide those of ordinary skill in the art with a complete disclosure and description of how the compounds, compositions, articles, devices and/or methods claimed herein are made and evaluated, and are intended to be purely exemplary of the invention. Efforts have been made to ensure accuracy.

### Example 1. Particle synthesis and dye coupling

Hydrogel particles capture particles containing novel affinity bait can be synthesized as both core and core-shell structures. The outer shell, included in the core-shell structure allows for size-sieving of higher molecular weight proteins in complex biofluids. In the subject invention both the core and the shell can contain the immobilized dye molecules or derivatives thereof.

### Poly(NIPAm-co-AAc) Core particle synthesis

N-isopropylacrylamide (NIPAm; Sigma-Aldrich) (4.750 g, 0.042 mol), N,N methylenebis-acrylamide (BIS Sigma-Aldrich;) (0.400 g, 0.0026mol), and Acrylic acid (AAc; Sigma-Aldrich); (.525 µL), 0.0073 mol) were dissolved in 500 mL of H₂O and then filtered using a nitrocellulose membrane disc filter (pore size 0.45 µm, Millipore) and transferred in a three neck round bottom flask. The solution was purged with nitrogen for 1 hour at room temperature , at medium stir rate, and then heated to 70°C. The basis for this specific step in the polymerization method of the poly(NIPAm-co-AA) core can be found elsewhere. Potassium Persulfate (KPS, Sigma-Aldrich) (0.276 g, 0.001mol) was dissolved in 5 mL of H₂O and was added to the solution to initiate polymerization. The reaction was maintained at 70°C under nitrogen for 6 h. Particles were washed five times via centrifugation (19000 rpm, 50 minutes, at Room Temperature) to eliminate un-reacted monomer and then re-suspended in 600 ml of H₂O.

### Poly(NIPAm-co-AAc) Core-Shell particle synthesis

N-isopropylacrylamide (NIPAm;Sigma-Aldrich) (1.55 g, 0.013 mol), ,bisacrylamide (BIS; Sigma-Aldrich) (162 mg,0.0010 mol) and KPS(0.092 g,,0.0003 mmol) were dissolved in 150 mL of H₂O , and then passed through a 0.45 µm filter. The solution was purged with nitrogen for 2 h at room temperature and medium stir rate. 200 mL of p-NIPAm-co-AAc core particles, were heated at 70°C and purged under nitrogen for 1 hour. Then 25 ml the solution containing NIPAm and BIS was added to the particles and allowed to react for 15 min at 70°C, after which the remaining 125 mL of shell solution was added in 25 mL aliquots over a period of two hours and 30 min. The reaction was maintained at 70°C under nitrogen for 3 hrs, then cooled overnight and washed as previously described. Washed particles were re-suspended in 200 mL of H₂O.
Synthesis of NIPAm particles, functionalized with amino-dyes-baits in water media. (Pigment Red 177; Orange 3; Pararosanilina; Acid blue 22; Acid black 48; Remazol brilliant blue; Alizarin blue black B; Brilliant Blue; Toluidine Blue; Rhodamine 123; Disperse yellow 3; Disperse Blue 3).

Dye molecules containing primary amine group were coupled via condensation to the carboxylic group of acrylic acid group, bonded covalently to NIPAm particles.
10 mL of NIPAm /AAc particles were centrifuged (16.1 rcf,25 C, 15 minutes) and re-suspended in 10 mL of NaH₂PO₄ at pH 5. The particles, 1 mL of SDS 1% (w/v), 824 mg of EDC (N-(3Dimethylaminopropyl)N' ethyl carbodiimide hydrochloride; Fluka Analytical) and 612 mg of NHS (N-Hydroxy succinimide; Sigma-Aldrich) were mixed in a three-neck round bottom flask, and allowed to react at room temperature and medium stir rate. After 15 min the reaction was stopped by centrifugation and re-suspension in 20 mL of Na₂HPO₄ 0.2 M (pH > 8). An excess amount of dye was dissolved in 180 mL Na₂HPO₄ 0.2 M, filtered via a 0.22 µm CA filter (Corning, NY, USA) and added to the activated particles, the reaction was maintained at room temperature at a medium stir rate overnight. The amount of dye required for the reaction was calculated so that the ratio of dye moles/AAc moles was 10:1, while the number of acrylic acid moles was 0.0000121. Figure 3a-3c indicates the amount of dye used for the reaction. Particles were washed with water to eliminate un-reacted dye followed by centrifugation at 16.1 rcf, 25uC, 15 minutes. Supernatant was discarded and particles resuspended in 10 mL of water.

Synthesis of NIPAm particles 13.80% AAc, functionalized with amino-dyes-baits in Dimethylformamide (DMF) media.

### (Orange 3; Pararosanilina; Disperse yellow 9,Pigment Red 177)

Amino dyes were coupled with NIPAm/AAcNP by phosponium/uranium activation in Dimethylformamide media.

10 mL of NIPAm /AAc was freeze-dried and re-suspended in 10 mL DMF (Dimethylformamide; Sigma-Aldrich). The particle solution was purged with nitrogen for 15 min at room temperature and medium stir rate. Then 68.84 mg of HBTU, 24.52 mg of HOBT and 40 µL of Nmm (N-Methylmopholine; Fluka Biochemika) were added to the particle solution and allowed to react at room temperature at medium stir rate for 5 minutes (the amount of HBTU (Peptides International) and HOBt (GL Biochem, Shanghai) was calculated considering a ratio reagent /AAc of 1.5, while the number of acrylic acid moles was 0.0000121). The reaction was maintained under nitrogen. An excess amount of dye was dissolved in DMF and added to the three-neck round bottom flask. The amount of each dye required for the reaction was calculated so that the ratio of dye moles/AAc moles was 10:1. The reaction was maintained under nitrogen for 6 hours. Particles were washed five times with decreasing concentrations of DMF, to eliminate un-reacted dye and to favor particle re-hydration, via centrifugation at 16.1 rcf, 25uC, 15 minutes. Supernatant was discarded and particles were re-suspended in 10 mL of water

### Synthesis of NIPAm/ Vinyl Sulfonic Acid Particles

N-isopropylacrylamide (NIPAm; Sigma-Aldrich) (0.475 g, 0.0042 mol), N,N methylenebis-acrylamide (BIS Sigma-Aldrich;) (0.040 g, 0.026mol), and Vinyl Sulfonic Acid (VSA; Sigma-Aldrich); (.80.4 µL), 0.00073 mol) were dissolved in 60 mL of H₂O and then filtered using a nitrocellulose membrane disc filter (pore size 0.45 µm, Millipore) and transferred in a three neck round bottom flask. The solution was purged with nitrogen for 1 hour at room temperature , at medium stir rate, and then heated to 70°C. KPS (0.028 g, 0.0001mol) was dissolved in 2.5 mL of H₂O and was added to the solution to initiate polymerization. The reaction was maintained at 70°C under nitrogen for 6 h. Particles were washed five times as previously described and then re-suspended in 60 ml of H₂O.
**Synthesis of boronic acid containing particles.** Particles containing boronic acid were obtained by precipitation polymerization. 0.93 g of NIPAm and 0.028 g of BIS were dissolved in 30 mL of water, vacuum filtered and thoroughly purged with nitrogen. 0.066 g of vinylphenyl boronic acid (VPBA) was dissolved in 2 mL of ethanol and added to the water solution, which was further purged with nitrogen under constant stirring. Temperature was raised to 75°C and kept constant for 15 minutes. 0.005 g of potassium persulfate (KPS) was added to the solution to initiate the reaction. The reaction was maintained at 75°C for 4 hours under nitrogen and continuous stirring. Particles were studied with light scattering and showed temperature and pH sensitivity suggesting the formation of hydrogel and the incorporation of VPBA. Boronic acid content of the particles will be quantified via the carmine assay.

### Example 2. Dyes bind different low abundance protein by SDS PAGE.

As shown in Figure 2a-2b specific classes of dyes with specific chemical side chain substitutions preferentially bind clinically relevant analytes. Some dye classes bind a limited set of analytes while others have larger spectrum of binding affinities. The chemical structure and the choice of the substitutions strongly determine the binding affinity and selectivity of the dye baits.

Aliquots of particles were incubated with a set of clinical proteins shown in Figure 2a-2b for 30 minutes room temperature. After incubation, the particles were centrifuged (7 min, 25 °C, 16 100 rcf), the supernatant was saved and the particles were washed three times by resuspending the pellets in 1 mL of water and centrifuging (7 min, 25 °C, 16 100 rcf). The particles and supernatants were then resuspending in 2% sample buffer and loaded directly on a 4-20% Tris-Glycine gel. The particles were retained in the stacking region of the gel while the proteins were electro-eluted and resolved in the gel. Proteins were detected using silver staining.

Hydrogel particles containing the following dyes: 1 Alizarin Blue Black B; 2 Disperse Blue 3; 3 Remazol Brilliant Blue R; 4 Pigment Red 177; 5 Acid Black 48; 6 Disperse Yellow 3; 7 Naphtol Blue Black B (Ab1); 8 Disperse Orange 3; 9 Disperse Yellow 9; 10 Rhodamine 123; 11 Toluidine Blue O; 12 Acrylic Acid, 13 Pararosaniline Base, 14 Brilliant Blue R; 15 Acid Blue 22, 16 Vinyl Sulfonic Acid; 17 Cibacron Blue F3GA were used for the SDS page binding studies (Figure 2a-2b). A summary of the protein binding was used to characterize the resulting binding. Complete binding resulted when all of the specific protein of interest was captured and eluted from the particle. Partial binding resulted when a fraction of the protein remained in the supernatant while the remaining fraction of the protein was captured by the particles. "Not-captured" resulted when the particles did not capture any protein. All of the protein remained in the supernatant.

### EXAMPLE 3. Particles increase the sensitivity of clinical grade immunoassay.

Aliquots of particles were incubated with aliquots of synthetic urine and synthetic sweat for 30 minutes room temperature under slow rotation. After incubation, the particles were centrifuged (7 min, 25 °C, 16 100 rcf), the supernatant was saved and the particles were washed three times by resuspending the pellets in 1 mL of water and centrifuging (7 min, 25 °C, 16 100 rcf). The particles were then incubated with elution buffers (70% acetonitrile, 10% ammonium hydroxide). Incubations with 30 mL of Surine were performed with 50 mL centrifuge tubes (Nalgene) and particles were separated from urine by centrifugation (45 min, 25 °C, 18 000 revolutions per minute (rpm)) and washed as described above.

### Elution of captured analytes from the particles

Hydrogel affinity bait capture particles can harvest target analytes, as demonstrated for a variety of analytes, a variety of chemical baits, and a variety of biologically relevant fluids such as urine, sweat and serum.

The washed pellet of particles was incubated for 15 min at room temperature with proper amounts of elution buffers. After incubation, the particles were centrifuged (7 min, 25 °C, 16 100 rcf) and the eluate was saved. The elution step was repeated twice and the eluates were pooled together. Eluate was freeze-dried.

The concentration of hGH eluted from particles was measured using the Immulite 1000 Growth Hormone System (Siemens Medical Solution Diagnostic). Eluates were diluted in GH Sample Diluent, and assayed according to the manufacturer's instructions.

### Results

Aliquots of 1 mL of synthetic urine containing human growth hormone at a concentration of 8.7 ng/mL were incubated with 1 mL of N-isopropylacrylamide/acid black 48. Particles were eluted with 70% acetonitrile-10% ammonium hydroxide and the eluted proteins were resuspended in the same volume as the initial solution (1 mL). The supernatant was completely depleted from the target analyte (hGH). The overall yield of the process approached (95%). (Figure 1)

In order to verify that the efficiency of the capture -elution process was not dependent on one particular analyte and one particular body fluid, 50 µL of artificial perspiration spiked with interlukin-6 at a concentration of 7912.5 pg/mL were processed with 50 µL of N-isopropylacrylamide/disperse yellow 9 particles (1 mg/mL). Proteins were eluted with 70% acetonitrile-10% ammonium hydroxide and the eluted proteins were resuspended in the same volume as the initial solution. The overall yield for this process was greater than 95%. (Figure 2a-2b)

N-isopropylacrylamide - Acid black 48 particles increase the sensitivity of a clinical grade immunoassay (Immulite 1000). Human growth hormone was spiked in 10 mL of synthetic urine and the solution was incubated with 1 mL of acid black 48 functionalized particles. Particles were separated by centrifugation and washed with water. Proteins were chemically eluted and freeze dried. Proteins were resuspended in a volume of 300 µL and analyzed with Immulite 1000. The concentration factor achieved exceeded 30x the starting concentration (Figure 3a-3c).

Remazol Brilliant Blue-R particles were incubated with 30 mL of synthetic urine containing human growth hormone at a concentration of 0.242 ng/mL. Proteins eluted from the washed particles were resuspended in 300 mL. The concentration factor achieved was higher than 60 times (Figure 4a-4b).

### EXAMPLE 4. Mass Spectrometry - Bait loaded particle incubation with serum.

The novel bait chemistries can be used to sequester, concentrate and preserve low abundance serum proteins. Application of the subject invention to serum biomarker discovery using mass spectrometry shows utility for the discovery of a large set of proteins of diagnostic relevance that were previously unknown to exist in blood.

Aliquots of 500 µL of serum were diluted 1:2 with 50 mM TrisHCl pH 7 and incubated with 500 µL of bait loaded particles listed in Figure 2a-2b. Particle-serum incubation time was 30 minutes. Particles were separated by centrifugation (16.1 rcf, 25 C, 10 minutes) and washed with 1 mL of 0.5 X PBS. After centrifugation (16.1 rcf, 25 C, 10 minutes) the supernatant was discarded and the pellet resuspended in 0.5 X PBS / 20% acetonitrile. The suspension was centrifuged again (16.1 rcf, 25 C, 10 minutes) and the supernatant discarded. The pellet was incubated for 5 minutes at 100 °C with 600 µL of elution buffer constituted by 1% TFA, 0.1 M Urea 80% ACN and then centrifuged (16.1 rcf, 25 C, 10 minutes). The elution step was repeated twice and the eluates were combined.

NIPAm/CB particles were washed twice with 0.25M NaSCN and eluted twice with 70% ACN, 10% NH4OH.

### NanoRPLC-MS/MS analysis

Eluates from the particles were analyzed by mass spectrometry. Proteins dried with SpeedVac were reconstituted in 8 M urea, reduced by 10 mM DTT, alkylated by 50 mM iodoacetamide, and digested by trypsin at 37 °C overnight. Tryptic peptides were further purified by Zip-Tip (Millipore) and analyzed by reversed-phase liquid chromatography nanospray tandem mass spectrometry (LC-MS/MS) using an LTQ-Orbitrap mass spectrometer (ThermoFisher). After sample injection by autosampler, the C₁₈ column (0.2×50mm, Michrom Bioresources, Inc.) was washed for 2 minutes with mobile phase A (0.1% formic acid) and peptides were eluted using a linear gradient of 0% mobile phase B (0.1% formic acid, 80% acetonitrile) to 50% mobile phase B in 50 minutes at 500 nanoliter/min, then to 100% mobile phase B for an additional 5 minutes. The LTQ mass spectrometer was operated in a data-dependent mode in which each full MS scan was followed by five MS/MS scans where the five most abundant molecular ions were dynamically selected for collision-induced dissociation (CID) using a normalized collision energy of 35%. Tandem mass spectra were searched against NCBI human database with SEQUEST using tryptic cleavage constraints. High-confidence peptide identifications were obtained by applying the following filter criteria to the search results: Xcorr versus charge >= 1.9, 2.2, 3.5 for 1+, 2+, 3+ ions; ΔCn > 0.1; probability of randomized identification <= 0.01.

### Results

Particles carrying the following baits were screened: 1 Alizarin Blue Black B; 2 Disperse Blue 3; 3 Remazol Brilliant Blue R; 4 Pigment Red 177; 5 Acid Black 48; 6 Disperse Yellow 3; 7 Naphtol Blue Black B (Ab1); 8 Disperse Orange 3; 9 Disperse Yellow 9; 10 Rhodamine 123; 11 Toluidine Blue O; 12 acrylic Acid, 13 Pararosaniline Base, 14 Brilliant Blue R; 15 Acid Blue 22, 16 Vinyl Sulfonic Acid; 17 Cibacron Blue F3GA (3). Aliquots of 100 µL of particles were incubated with 100 µL of serum diluted 1:2 with 50 mM Tris HCl pH 7. Particles were allowed to incubate with serum for 30 minutes and then separated by centrifugation. Protein captured by the particles were chemically eluted, trypsin digested and sequenced by mass spectrometry.

Results reported in Figure 4a-4b demonstrated that most of the dyes functionalized particles allowed sequencing of new proteins never sequenced in serum before (not present in the HUPO Plasma Proteome Project comprehensive list, http://www.hupo.org/research/hppp/)

Particles carrying the following chemical affinity bait remazol brilliant blue R, disperse yellow 3, disperse yellow 9, acrylic acid, cibacron blue F3GA, and mix = mixture of remazol brilliant blue R, disperse yellow 3, and disperse yellow 9 were selected. Aliquots of 500 µL of particles (1 mg/mL) were incubated with 500 µL of serum for 30 minutes, separated by centrifugation and chemically eluted. Proteins were trypsin digested and sequenced by mass spectrometry (Figure 5- 14)

Results demonstrated that particles carrying different dyes bind different groups of proteins. Protein sequestration is dependent on the chemical structure of the affinity bait.

### EXAMPLE 5 Cibacron Blue F3GA core - Vinylsulfonic acid shell particle preparation.

Core shell bait containing particles have been prepared which contain two different classes of dye bait in the shell and the core. This double bait design provides a novel capture affinity profile. Unwanted contaminating albumin is EXCLUDED by the dye in the outer shell while at the same time the desired analyte is CAPTURED by the core (Figure 5).

N-isopropylacrylamide (NIPAm)/Allylamine (AA) particles were prepared containing 10% of AA with respect to the total monomer. NIPAm (0.89 g, 7.83 mmol) and N,N'-methylenebisacrylamide (BIS, 0.042 g, 0.27 mmol) were dissolved in 30 mL of water and then passed through a 0.2 µm filter nylon membrane. The solution was purged with nitrogen for 15 min at room temperature with a medium stirring rate before AA (0.051 g, 0.90 mmol) was added. The solution was purged with nitrogen for another 15 min and then heated to 75 °C. KPS (0.0070 g, 0.025 mmol) in 1.0 mL of water was added to the solution to initiate polymerization. The reaction was maintained at 75°C under nitrogen for 3 hours. The react ion was subsequently allowed to cool to room temperature and stirred overnight. The reaction mixture was then transferred to 2 mL microcentrifuge tubes and the particles harvested and washed via centrifugation (Eppendorf 5415R centrifuge). After centrifuging the particle suspension for 20 min at 23 °C and 16 100 relative centrifugal force (rcf), the supernatant from each tube was decanted and the particles were redispersed in 1.0 mL water. This concentration/redispersion process was repeated for a total of five washes. To obtain NIPAm/Cibacron Blue F3GA (CB) particles, CB (0.76 g, 0.90 mmol) was dissolved in 10 mL of 0.1 mol/L aqueous sodium carbonate. The NIPAm/AA particle suspension (10 mL volume) was purged with nitrogen for 15 min with a medium stirring rate in a 100 mL three-neck round-bottom flask, after which solid sodium carbonate (0.106 g, 1.0 mmol) was added to the suspension. The suspension was then stirred at room temperature under nitrogen for about 1 min. The CB solution was then added to the NIPAm/ AA particle suspension, and the combined reaction mixture was then stirred at room temperature under nitrogen for 48 h. The resulting NIPAm/CB particles were harvested and washed using centrifugation as described earlier in the preparation of NIPAm/ AA particles. Dye loading was determined via spectrophotometry (Thermo Spectronic 20+). A calibration curve was constructed using CB stock solutions of known concentrations. The supernatants from the entire concentration/redispersion process were combined, and their absorbance at a wavelength of 608 nm was determined. The CB concentration of the combined supernatants was estimated using the calibration curve. NIPAm/CB particle diameter at room temperature is 312 nm, as measured with Submicron Particle Size Analyzer, Beckman Coulter. The measurement of particle diameter was performed using water as diluent (refractive index (RI) = 1.333, diluent viscosity=0.890 cP). The test angle was 90°. Average values were calculated for three measurements using a 200 s integration time, and the solutions were allowed to thermally equilibrate for 10 min before each set of measurements. Measured values were then converted to particle sizes via the Stokes Einstein relationship.

In order to build a vinylsulfonic acid (VSA) shell on the NIPAm/CB core, 20 mL of NIPAm/CB particle suspension, obtained as described before, were heated at 70 °C and purged with nitrogen for 1 hour. NIPAm (0.156 g, 1.38 mmol), BIS (0.013 g, 0.084 mmol), vinylsulphonic acid (26 ul, 0.334 mmol) and KPS (0.092 g, 0.328 mmol) were dissolved in water and passed through a 0.2 µm filter nylon membrane. After one hour, 3 mL of this solution were added to NIPAm/CB particle suspension and the remaining 17 mL of solution were added in aliquots of 3 mL every five minutes. Particle diameter after the shell synthesis reaction was 368 nm, as measured with Submicron Particle Size Analyzer.

### Results:

In order to prove the utility of a structure comprising two different baits in the core and in the shell compartment, particles with comprised of cibacron blue F3GA core and containing vinyl sulfonic acid shell were obtained. Particles with the following properties were experimentally compared: vinylsulfonic acid core particles, cibacron blue F3GA particles, cibacron blue F3GA - N-isopropylacrylamide shell, and cibacron blue F3GA core - vinyl sulfonic acid shell. Aliquots of 100 µL of particles were incubated with 10 µL of serum, separated by centrifugation and washed two times with water. Particles were loaded on a 4-20% TrisGly gel. SDS PAGE analysis revealed that: 1) vinyl sulfonic acid functionalized particles do not uptake significant amount of albumin (lane 2), 2) cibacron blue F3GA particles have a different and richer pattern of captured proteins with respect to the vinylsulfonic acid particles and capture a certain amount of albumin (lane 3), 3) cibacron blue F3GA core- N-isopropylacrylamide shell particles maintain similar behavior to cibacron blue F3GA core particles (lane 4), 4) cibacron blue F3GA core - vinylsulfonic acid shell particles do not uptake significant amount of albumin and maintain a similar pattern of harvested low molecular proteins as cibacron blue F3GA core. Therefore, particles containing two different baits in the core and in the shell acquire a behavior that is the sum of the characteristic of the two baits. In particular in this embodiment of the invention, vinyl sulfonic acid shell did not prevent low molecular weight proteins from interacting with cibacron blue F3GA core.

### EXAMPLE 6

### Influence of the chemical bait structure on affinity of particles towards protein analytes.

In order to investigate the effect of the chemical structure of the affinity bait on the binding properties of particles, structure number 5 (acid black 48) was selected as a starting structure. Acid black 48 contains two anthraquinone rings and a number of sulfonate side groups. Structures 1 to 4 were selected for their similarity to acid black 48. Structures 1-4 contain anthraquinone rings, sulfonate substitution and other substitution groups such as methyl groups (structure 2), aryl groups (structure 1 and 3) or no substitution groups (structure 4). Particles carrying vinylsulfonic acid are labeled with number 16 in Figure 6. Particles carrying the baits described above were incubated with low abundance, low molecular weight protein analytes and analyzed by SDS PAGE. Results showed that changes in the structure of the chemical bait affect binding properties of the particles such that particles functionalized with different baits have different binding patterns. For example, the chemokine Eotaxin 2 was completely captured by particles containing structure number 1, 4, and 5, was partially associated to particles carrying structure 2 and was not captured by particles carrying structure 3.

## Claims

1. A chemical affinity bait, comprising: a dye affinity bait covalently immobilized within a hydrogel particle matrix structure, **characterised in that** the dye affinity bait comprises chemical dye molecules including monazo, diazo, polyazo, anthraquinone, triphenylmethane, xanthenes and indigo dye classes.

2. A chemical affinity bait as claimed in claim 1, wherein analyte enrichment and isolation are conducted by particles ranging in size from 1 nm to 100µm.

3. A chemical affinity bait as claimed in claim 1, further comprising particles comprised of polymers, peptides, proteins, carbohydrates and inorganic porous particles.

4. A chemical affinity bait as claimed in claim 3, wherein the inorganic porous particles are of silica, titanium dioxide, alumina.

5. A chemical affinity bait as claimed in claim 1, wherein said dye affinity bait comprises anthraquinone dyes with the general formula (a): 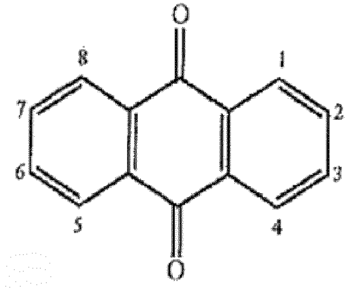 wherein locations 1-8 represent bond locations for hydroxyl group, halogen groups, sulfonyl groups, alkyl groups, benzyl groups, amino groups, carboxy groups, cyano groups, or phosphorous groups.

6. A chemical affinity bait as claimed in claim 1, wherein said dye affinity bait comprises: reactive dyes with the general formula (b): 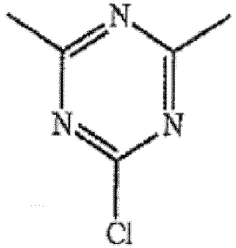 wherein R and R' are independently selected from halogen groups and from substituted and unsubstituted aryl amine groups.

7. A chemical affinity bait as claimed in claim 6, wherein the aryl amine groups may be substituted with hydroxyl, carbonyl, sulfonic and/or alkyl groups.

8. A chemical affinity bait as claimed in claim 1, wherein said dye affinity bait comprises: aryl methane dyes with the general formula (c): 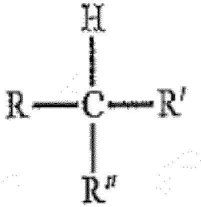 wherein R, R', and R" are independently selected from substituted and unsubstituted aryl groups; optionally phenyl, naphthyl, anthracenyl.

9. A chemical affinity bait as claimed in claim 8, wherein the aryl groups are substituted with amino, hydroxyl, carbonyl, sulfonic and/or alkyl groups.

10. A chemical affinity bait as claimed in claim 1, wherein said dye affinity bait comprises: aromatic azo dyes with the general formula (d):
X-R₁-N=N-R₂-Y
wherein R₁ is an aromatic group and R₂ is selected from the group consisting of aliphatic and aromatic groups, and X and Y are independently selected from the groups consisting of hydrogen, halides, - NO₂, -NH₂, aryl groups, alkyl groups, alkoxy groups, sulfonate groups, -SO₃H, -OH, -COH, -COOH.

11. A chemical affinity bait as claimed in claim 1, wherein said dye affinity bait comprises azoxy compound X-R₁-N=NO-R₂-Y or hydrazo compound X-R₁-NH-NH-R₂-Y, wherein R₁ is an aromatic group and R₂ is selected from the group consisting of aliphatic and aromatic groups, and X and Y are independently selected from the groups consisting of hydrogen, halides, -NO₂, -NH₂, aryl groups, alkyl groups, alkoxy groups, sulfonate groups, -SO₃H, -OH, -COH, -COOH.

12. A chemical affinity bait as claimed in claim 1, wherein said dye affinity bait comprises dyes with the general formula (e): 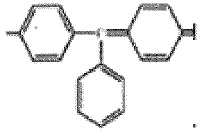 preferably wherein groups substituted on the fused ring structure include hydroxyl, halogen, chlorine, bromine, sulfonyl, sulfonic acid salts, alkyl, benzyl, primary amine, secondary amine, tertiary amine, quaternary amine, carboxy, cyano or phosphorous.

13. A chemical affinity bait as claimed in claim 1, wherein dye affinity bait comprises: affinity ligand-matrix conjugates comprising heterocyclic fused rings with the general formula (f): 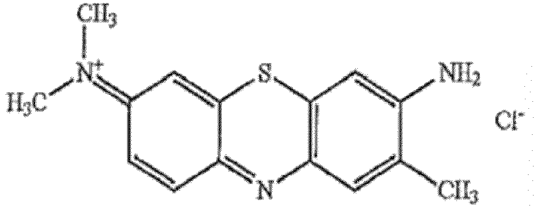 wherein substitutions on the fused ring structure include sulfur, oxygen, nitrogen, or carbon; or groups selected from hydroxyl, primary amine, secondary amine, tertiary amine, quaternary amine, hydrogen, -NO₂, -NH₂, aryl, alkyl, alkoxy, sulfonate, -SO₃H, -COH, -COOH or halide.

## Patentansprüche

1. Chemischer Affinitätslockstoff, umfassend: einen Farbstoffaffinitätslockstoff, der in einer Hydrogelpartikelmatrixstruktur kovalent immobilisiert ist, **dadurch gekennzeichnet, dass** der chemische Affinitätslockstoff chemische Farbstoffmoleküle umfasst, welche die Farbstoffklassen Monazo, Diazo, Polyazo, Anthrachinon, Triphenylmethan, Xanthene und Indigo aufweisen.

2. Chemischer Affinitätslockstoff nach Anspruch 1, wobei Analytanreicherung und -isolierung durch Teilchen mit Größen zwischen 1 nm und 100 µm durchgeführt wird.

3. Chemischer Affinitätslockstoff nach Anspruch 1, ferner Teilchen umfassend, die aus Polymeren, Peptiden, Proteinen, Kohlehydraten und anorganischen porösen Teilchen bestehen.

4. Chemischer Affinitätslockstoff nach Anspruch 3, wobei die anorganischen porösen Teilchen aus Kieselerde, Titandioxid, Aluminiumoxid bestehen.

5. Chemischer Affinitätslockstoff nach Anspruch 1, wobei der Farbstoffaffinitätslockstoff Anthrachinon-Farbstoffe mit der allgemeinen Formel (a) umfasst: 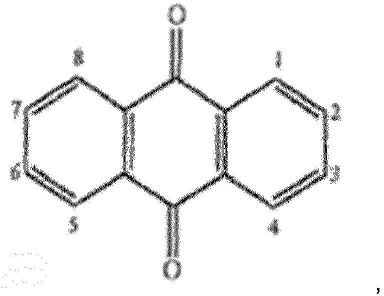 wobei die Stellen 1-8 Bindungsstellen für Hydroxylgruppen, Halogengruppen, Sulfonylgruppen, Alkylgruppen, Benzylgruppen, Aminogruppen, Carboxygruppen, Cyanogruppen oder Phosphorgruppen darstellen.

6. Chemischer Affinitätslockstoff nach Anspruch 1, wobei der Farbstoffaffinitätslockstoff folgendes umfasst:
Reaktivfarbstoffe mit der allgemeinen Formel (b): 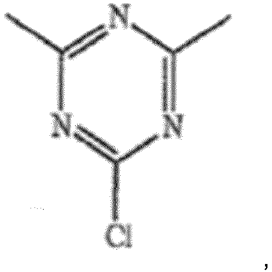 wobei R und R' unabhängig ausgewählt sind aus Halogengruppen und aus substituierten oder nicht substituierten Arylamingruppen.

7. Chemischer Affinitätslockstoff nach Anspruch 6, wobei die Arylamingruppen mit Hydroxyl-, Carbonyl-, Schwefel- und/oder Alkylgruppen substituiert sein können.

8. Chemischer Affinitätslockstoff nach Anspruch 1, wobei der Farbstoffaffinitätslockstoff folgendes umfasst: Arylmethanfarbstoffe mit der allgemeinen Formel (c): 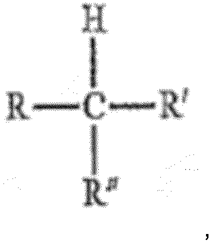 wobei R, R' und R" unabhängig ausgewählt sind aus substituierten oder nicht substituierten Arylgruppen; optional Phenyl, Naphthyl, Anthracenyl.

9. Chemischer Affinitätslockstoff nach Anspruch 8, wobei die Arylgruppen mit Amino-, Hydroxyl-, Carbonyl-, Schwefel- und/oder Alkylgruppen substituiert sind.

10. Chemischer Affinitätslockstoff nach Anspruch 1, wobei der Farbstoffaffinitätslockstoff folgendes umfasst:
aromatische Azo-Farbstoffe mit der allgemeinen Formel (d):
X-R₁-N=N-R₂-Y,
wobei R₁ eine aromatische Gruppe ist, und wobei R₂ ausgewählt ist aus der Gruppe bestehend aus aliphatischen und aromatischen Gruppen, und wobei X und Y unabhängig ausgewählt sind aus den Gruppen bestehend aus Wasserstoff, Haliden, -NO₂, -NH₂, Arylgruppen, Alkylgruppen, Alkoxygruppen, Sulfonatgruppen, -SO₃H, -OH, -COH, -COOH.

11. Chemischer Affinitätslockstoff nach Anspruch 1, wobei der Farbstoffaffinitätslockstoff eine Azoxyverbindung X-R₁-N=NO-R₂-Y oder eine Hydrazoverbindung X-R₁-NH-NH-R₂-Y umfasst, wobei R₁ eine aromatische Gruppe ist und R₂ ausgewählt ist aus der Gruppe bestehend aus aliphatischen und aromatischen Gruppen, und wobei X und Y unabhängig ausgewählt sind aus den Gruppen bestehend aus, Haliden, -NO₂, -NH₂, Arylgruppen, Alkylgruppen, Alkoxygruppen, Sulfonatgruppen, -SO₃H, -OH, -COH, -COOH.

12. Chemischer Affinitätslockstoff nach Anspruch 1, wobei der Farbstoffaffinitätslockstoff Farbstoffe mit der allgemeinen Formel (e) umfasst: 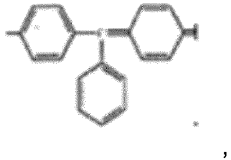 wobei an der verbundenen Ringstruktur substituierte Gruppen vorzugsweise Hydroxyl, Halogen, Chlor, Brom, Sulfonyl, Schwefelsäuresalze, Alkyl, Benzyl, primäres Amin, sekundäres Amin, tertiäres Amin, quaternäres Amin, Carboxy, Cyano oder Phosphor aufweisen.

13. Chemischer Affinitätslockstoff nach Anspruch 1, wobei der Farbstoffaffinitätslockstoff folgendes umfasst: Affinitätsliganden-Matrix-Konjugate, die heterocyclisch verbundene Ringe mit der allgemeinen Formel (f) umfassen: 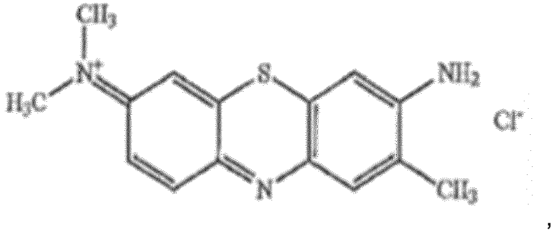 wobei Substitutionen an der verbundenen Ringstruktur Schwefel, Sauerstoff, Wasserstoff oder Kohlenstoff aufweisen; oder Gruppen, die ausgewählt sind aus Hydroxyl, primärem Amin, sekundärem Amin, tertiärem Amin, quaternärem Amin, Wasserstoff, -NO₂, -NH₂, Aryl Alkyl, Alkoxy, Sulfonat, -SO₃H, -COH, -COOH oder Halid.

## Revendications

1. Appât d'affinité chimique, comprenant : un appât d'affinité à un colorant immobilisé par covalence dans une structure de matrice de particules d'hydrogel, **caractérisé en ce que** l'appât d'affinité à un colorant comprend des molécules de colorant chimique comprenant des classes de colorant monazo, diazo, polyazo, anthraquinone, triphénylméthane, xanthènes et indigo.

2. Appât d'affinité chimique selon la revendication 1, l'enrichissement et l'isolement de la substance à analyser étant effectués par des particules dont la taille varie de 1 nm à 100 µm.

3. Appât d'affinité chimique selon la revendication 1, comprenant en outre des particules constituées de polymères, peptides, protéines, hydrates de carbone et particules poreuses inorganiques.

4. Appât d'affinité chimique selon la revendication 3, les particules poreuses inorganiques étant en silice, dioxyde de titane, alumine.

5. Appât d'affinité chimique selon la revendication 1, ledit appât d'affinité à un colorant comprenant des colorants anthraquinoniques de formule générale (a) : 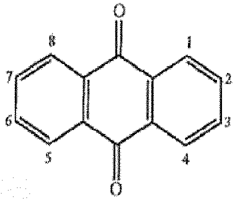 les emplacements 1-8 représentant des emplacements de liaison pour des groupes hydroxyle, des groupes halogène, des groupes sulfonyle, des groupes alkyle, des groupes benzyle, des groupes amino, des groupes carboxy, des groupes cyano ou des groupes phosphore.

6. Appât d'affinité chimique selon la revendication 1, ledit appât d'affinité à un colorant comprenant des colorants réactifs de formule générale (b) : 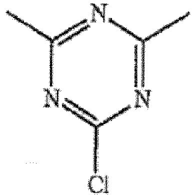 où R et R' sont choisis indépendamment parmi les groupes halogène et les groupes arylamine substitués et non substitués.

7. Appât d'affinité chimique selon la revendication 6, les groupes arylamine pouvant être substitués par des groupes hydroxyle, carbonyle, sulfonique et/ou alkyle.

8. Appât d'affinité chimique selon la revendication 1, ledit appât d'affinité à un colorant comprenant : des colorants arylméthane de formule générale (c) : 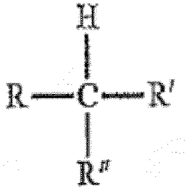 où R, R' et R" sont choisis indépendamment parmi les groupes aryle substitués et non substitués ; éventuellement phényle, naphtyle, anthracényle.

9. Appât d'affinité chimique selon la revendication 8, les groupes aryle étant substitués par des groupes amino, hydroxyle, carbonyle, sulfonique et/ou alkyle.

10. Appât d'affinité chimique selon la revendication 1, ledit appât d'affinité à un colorant comprendant : des colorants azoïques aromatiques de formule générale (d) :
X-R₁-N=N-R₂-Y
où R₁ est un groupe aromatique et R₂ est choisi dans le groupe constitué des groupes aliphatiques et aromatiques, et X et Y sont choisis indépendamment dans les groupes constitués par l'hydrogène, les halogénures, -NO₂, -NH₂, les groupes aryle, les groupes alkyle, les groupes alcoxy, les groupes sulfonate, -SO₃H, -OH, -COH, -COOH.

11. Appât d'affinité chimique selon la revendication 1, ledit appât d'affinité à un colorant comprenant le composé azoxy X-R₁-N=NO-R₂-Y ou le composé hydrazo X-R₁-NH-NH-R₂-Y, où R₁ est un groupe aromatique et R₂ est choisi dans le groupe constitué par les groupes aliphatique et aromatique, et X et Y sont indépendamment choisis dans les groupes constitués par l'hydrogène, les halogénures, -NO₂, -NH₂, les groupes aryle, alkyle, alcoxy et sulfonate, -SO₃H, -OH, -COH et -COOH.

12. Appât d'affinité chimique selon la revendication 1, ledit appât d'affinité à un colorant comprenant des colorants de formule générale (e) : 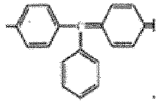 de préférence les groupes substitués sur la structure cyclique fusionnée comprenant les groupes hydroxyle, halogène, chlore, brome, sulfonyle, sels d'acide sulfonique, alkyle, benzyle, amine primaire, amine secondaire, amine tertiaire, amine quaternaire, carboxy, cyano ou phosphoreux.

13. Appât d'affinité chimique selon la revendication 1, l'appât d'affinité à un colorant comprenant : des conjugués ligand-matrice d'affinité comprenant des cycles hétérocycliques fusionnés de formule générale (f) : 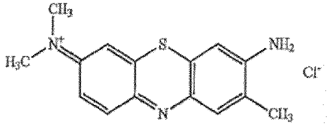 les substitutions sur la structure cyclique fusionnée comprenant le soufre, l'oxygène, l'azote ou le carbone ; ou des groupes choisis parmi les groupes hydroxyle, amine primaire, amine secondaire, amine tertiaire, amine quaternaire, hydrogène, -NO₂, -NH₂, aryle, alkyle, alcoxy, sulfonate, -SO₃H, -COH, -COOH ou halogénure.
